# EUROPÄISCHE PATENTSCHRIFT

(11) **EP 1 897 588 B1**
(45) Veröffentlichungstag und Bekanntmachung des Hinweises auf die Patenterteilung: **11.03.2009**
(21) Anmeldenummer: 07015665.8
(22) Anmeldetag: 09.08.2007
(51) Int. Cl.: A61N 1/375, H01G 4/35, H01M 2/06

(54) **Elektrische Durchführung**
Feedthrough connection
Conducteurs tranversants

(30) Priorität: 07.09.2006 DE 102006041939
(43) Veröffentlichungstag der Anmeldung: 12.03.2008
(73) Patentinhaber: BIOTRONIK CRM Patent AG, 6341 Baar (CH)
(72) Erfinder: Eck, Stefan, 91315 Höchstadt (DE); Frauenstein, Boris, 91074 Herzogenaurach (DE); Haas, Erich, 91604 Flachslanden (DE); Teske, Josef, 96103 Hallstadt (DE)
(74) Vertreter: Lindner-Vogt, Karin L.

(56) Entgegenhaltungen:
- DE-A1- 10 329 261
- US-A- 5 272 283
- US-A- 5 738 270
- US-A1- 2003 083 715

## Beschreibung

Die Erfindung betrifft eine elektrische Durchführung zum Einsetzen in eine Öffnung eines implantierbaren elektrischen Therapiegerätes. Solche elektrischen Therapiegeräte sind beispielsweise implantierbare Herzschrittmacher, implantierbare Kardioverter/Defibrillatoren oder Kochlea-Implantate.

Die elektrische Durchführung besitzt einen elektrisch isolierenden Isolationskörper, durch den wenigstens ein elektrisch leitender Anschlusspin hindurchtritt, der hermetisch dicht mit dem Isolationskörper verbunden ist.

Derartige elektrische Durchführungen dienen dazu, eine elektrische Verbindung zwischen einem hermetisch abgeschlossenen Inneren eines Therapiegerätes und dem Äußeren des Therapiegerätes herzustellen. Bei bekannten Elektrotherapiegeräten, wie beispielsweise Herzschrittmachern oder Kardioverter/Defibrillatoren, ist üblicherweise ein hermetisch dichtes Metallgehäuse vorgesehen, welches auf einer Seite einen Anschlusskörper, auch Header genannt, aufweist, der Anschlussbuchsen für das Anschließen von Elektrodenleitungen trägt. Die Anschlussbuchsen weisen elektrische Kontakte auf, die dazu dienen, Elektrodenleitungen elektrisch mit der Steuerelektronik im Inneren des Gehäuses des Herzschrittmachers zu verbinden. Dort, wo die elektrische Verbindung in das Gehäuse des Herzschrittmachers eintritt, ist üblicherweise eine Durchführung vorgesehen, die hermetisch dichtend in eine entsprechende Gehäuseöffnung eingesetzt ist.

Häufig sind derartige elektrische Durchführungen als Filterdurchführungen ausgebildet. In diesem Falle tragen die Vorrichtungen ein elektrisches Filter gegen hochfrequente elektromagnetische Strahlung, so dass entsprechende Signale der Steuerelektronik im Inneren des Gehäuses wenn überhaupt nur stark gedämpft zugeführt werden und die Steuerelektronik erst bei wesentlich größeren Signalstärken der elektrischen Störung gestört wird, als dies ohne elektrisches Filter der Fall wäre. Ein derartiges Tiefpass-Filter wird üblicherweise von einem Filterkörper gebildet, der nach Art eines Kondensators zwischen eine Gerätemasse und eine jeweilige, durch die Durchführung hindurchtretende, elektrische Leitung geschaltet ist.

Eine solche, durch die Durchführung hindurchtretende elektrische Leitung wird üblicherweise von einem elektrisch leitenden Anschlusspin gebildet, der durch eine Durchgangsöffnung in einem elektrisch isolierenden Isolationskörper hindurchtritt. Der elektrisch leitende Anschlusspin steht auf beiden Seiten über die jeweilige Stirnfläche des Isolationskörpers über, so dass auf beiden Seiten des Isolationskörpers - und damit auf beiden Seiten der elektrischen Durchführung - jeweils weiterführende elektrische Leitungen an den Anschlusspin - beispielsweise durch Löten oder Schweißen - angeschlossen werden können. Ein möglicher Spalt zwischen einer Durchgangsöffnung im Isolationskörper, durch den ein jeweiliger Anschlusspin hindurchtritt und dem Anschlusspin selbst, wird üblicherweise mit einem Lot, in der Regel Goldlot, hermetisch dicht verschlossen.

Eine Vielzahl derartiger elektrischer Durchführungen sind aus dem Stand der Technik bekannt. Beispiele sind in US 6,934,582, US 6,822,845, US 6,765,780, US 6,643,903, US 6,567,259, US 6,768,629, US 6,765,779, US 6,566,978 und US 6,529,103 zu finden.

In der US 5,272,283 ist offenbart, dass der Isolationskörper durch Sintern hergestellt ist und mindestes ein Anschlusspin durch dieses Sintern mit dem Isolationskörper hermetisch dicht verbunden ist. Der Anschlusspin ist also direkt beim Herstellen des Isolationskörpers durch Sintern in diesen eingebunden. Eine solche Konfiguration bedeutet zwar eine sicher hermetisch abdichtende Verbindung zwischen dem Isolationskörper und den Anschlusspins, jedoch können auch hier weitere Fehler beim Auschluss der Durchführung an das Therapiegerät auftreten.

Trotz der Vielzahl bekannter Durchführungen besteht nach wie vor der Bedarf, diese hinsichtlich Herstellbarkeit und Eigenschaften und Zuverlässigkeit zu verbessern.

Erfindungsgemäß wird dieses Ziel dadurch erreicht, dass die Durchführung einen Flansch aufweist, der in einem Sinterschritt mit dem Isolationskörper hergestellt und so zusammen mit dem mindestens einen Anschlusspin und dem Isolationskörper hermetisch dicht verbunden wird.

Der Flansch der Durchführung wiederum ist vorzugsweise mittels Verschweißen mit dem Metallgehäuse eines implantierbaren elektrischen Therapiegerätes hermetisch dicht verbunden.

Hieraus ergibt sich als Vorteil, dass die Anzahl der Komponenten und Prozessschritte bei der Herstellung reduziert ist und damit auch die Fehlerquote bei der Herstellung sinkt und die Zuverlässigkeit der Durchführung erhöht ist.

Ein weiterer wichtiger Vorteil ist, dass kein leitfähiges Lot wie beispielsweise Gold benötigt wird, um Pin und Flansch hermetisch dicht mit dem Isolationskörper zu verbinden. Leitfähiges Lot wie Goldlot erfordert nämlich mindestens zwei getrennte Lotreservoire, da es ansonsten zwischen Pin und Flansch zu einem elektrischen Kurzschluss kommen würde. Somit ermöglicht ein in einem einzigen Sinterschritt hergestellter und gleichzeitig mit Pin und Flansch verbundener Isolationskörper einfachere und kompaktere Konstruktionen von elektrischen Durchführungen.

Auch lässt sich auf diese Weise ohne weiteres eine biokompatible Oberfläche des Isolationskörpers auf seiner Außenseite (bezogen auf den eingebauten Zustand) erzielen.

Der letztgenannte Vorteil ist insbesondere dann gegeben, wenn der Isolationskörper aus einem keramischen Material besteht, welches vorzugsweise Al₂O₃ enthält. Besonders bevorzugt besteht der Isolationskörper aus einem glaskeramischen oder glasartigen Marerial.

Das Isolationsmaterial kann an seiner Oberfläche so beschichtet sein, dass sich auf seiner dem Körper zugewandten Seite ein biokompatibles Material befindet, welches vorzugsweise Al₂O₃ enthält.

Die Durchführung ist für Hochspannungsanwendungen wie bei Defibrillatoren besonders geeignet, wenn die Isolationskeramik so ausgeformt ist, dass lange Isolationsstrecken auf der Oberfläche und im Volumen entstehen. Geeignete Ausformungen sind z. B. Ausbuchtungen und Kanten. Solche Ausformungen werden vorzugsweise auf der körperzugewandten Seite der Durchführung ausgebildet.

Zudem bieten derartige Ausformungen stabile Verankerungsmöglichkeiten für den Header, so dass dessen Anbindung an das Gehäuse des Implantats sicherer wird.

Der Anschlusspin besteht vorzugsweise aus Metall, welches vorzugsweise Platin enthält und besonders bevorzugt eine Platin-Iridium-Legierung ist. Als weitere, besonders biokompatible und korrosionsbeständige Metalle für den Pin kommen Iridium, Niob, Tantal und Titan und deren geeignete Legierungen in Betracht. Derartige Anschlusspins weisen die gewünschte Biokompatibilität auf, sind korrosionsbeständig und lassen sich zuverlässig verarbeiten.

Um für Therapiegeräte geeignet zu sein, deren elektrische Komponenten im Inneren des Gehäuses über mehrere elektrische Leitungen, beispielsweise mit einer oder mehreren Elektrodenleitungen zu verbinden sind, ist die Durchführung vorzugsweise mehrpolig ausgebildet und weist hierzu mehrere, vorzugsweise parallel zueinander verlaufende Anschlusspins auf.

In einer bevorzugten Ausführungsvariante einer mehrpoligen Durchführung besitzt jeder Pin seinen separaten Isolationskörper mit dem Vorteil, dass die Isolationskörper rotationssymmetrisch, z. B. zylindrisch ausgebildet sein können und einfach herstellbar sind.

Das Anschließen von elektrischen Leitungen eines Headers wird erleichtert, wenn die Anschlusspins auf der Außenseite der Durchführung (bezogen auf den eingebauten Zustand) eine unterschiedliche Länge aufweisen. Das Anschließen der elektrischen Leitungen wird in machen Fällen weiter erleichtert, wenn die Pins an ihren Enden abgeflacht, gebogen, in Form von Nägelköpfen oder in sonstige geeignete Formen gebracht werden. Es können jedoch auch im eingebauten Zustand gleich lange Anschlusspins vorgesehen werden.

Um bei einem möglichst kleinen Isolationskörper einen möglichst großen Abstand der Anschlusspins voneinander zu erreichen, sind die Anschlusspins vorzugsweise parallel zueinander verlaufend auf einem konzentrisch zum Isolationskörper verlaufenden Kreisbogen gleichmäßig verteilt angeordnet. Alternativ können die Anschlusspins aber auch linear in einer Ebene im Isolationskörper angeordnet sein. Dies kann weitere Fertigungsschritte bei der Schrittmacherherstellung erleichtern. Auch eine lineare Anordnung, bei der zwei oder mehr Reihen von Anschlusspins jeweils zueinander versetzt im Isolationskörper angeordnet sind, kommt in Betracht.

Insbesondere bei der ersten der drei letztgenannten Ausführungsvarianten ist es vorteilhaft, wenn der Kreiskörper eine quer zur Längsrichtung des Anschlusspins oder der Anschlusspins verlaufende Querschnittsfläche aufweist, die rund und vorzugsweise kreisförmig ist.

Der Isolationskörper ist vorzugsweise quer zur Längsrichtung der Pins von einem hülsenartigen, metallischen Flansch eingefasst. Der Flansch besteht vorzugsweise aus einem Material, das bezüglich seiner Zusammensetzung weitgehend dem metallischen Gehäuse des Therapiegerätes gleicht. Der Flansch ist entweder aus einem Vollmaterial z. B. durch Drehen oder Fräsen herausgearbeitet oder auch selbst durch einen geeigneten Sinterprozess hergestellt. Im letztgenannten Fall kann der Flanschkörper von kleinen Poren durchsetzt sein, die jedoch nicht die Hermetizität des Flansches beeinträchtigen. Ein derartiger Flansch kann beispielsweise durch Schweißen mit einem metallischen Gehäuse des Therapiegerätes hermetisch dicht verbunden werden. Flansch und Isolationskörper sind gemäß einer bevorzugten Ausführungsvariante durch Sintern des Isolationskörpers hermetisch dicht miteinander verbunden.

Die Durchführung ist vorzugsweise als Filterdurchführung mit einem Filterkörper ausgebildet. Der Filterkörper besitzt parallel zueinander verlaufende, scheibenförmige Kondensatorelektroden, die abwechselnd mit dem Flansch und mit einem Anschlusspin elektrisch leitend verbunden sind.

Im Zusammenhang mit der letztgenannten Ausführungsvariante erstreckt sich der Flansch vorzugsweise soweit über die innere Stirnfläche des Isolationskörpers hinaus, dass der Flansch auch den Filterkörper wenigstens über den größten Teil seiner Länge umschließt und auf diese Weise leicht elektrisch leitend mit den Kondensatorelektroden des Filterkörpers zu verbinden ist.

Bestehen die Pins aus Iridium, Niob, Tantal, Titan oder ähnlichen, nicht direkt weich lötbaren Materialien, wird die elektrisch leitfähige Verbindung der Pins mit den Kondensatorelektroden des Filterkörpers über elektrisch leitfähige Kleber oder durch Weichlöten erheblich erleichtert, wenn die Pins mit Goldlot vergoldet sind. Die Vergoldungen können sich auf die Bereiche der Pins beschränken, die für die elektrische Anbindung der Pins mit den Kondensatorelektroden der Filterkörper entscheidend sind.

In einer Variante werden die Kondensatorelektroden des Filterkörpers mit den Pins und dem Flansch beispielsweise direkt mit Goldlot angelötet. Dazu bedarf es eines besonders wärmebeständigen Filterkörpers. Eine gefilterte Durchführung kann auf diese Weise kostengünstig in einem einzigen, kombinierten Löt-/Sinter-Schritt gefertigt werden. In diesem Fall kann die Anwendung von Gold- oder Glaskeramik-Lot entfallen, statt dessen sind die Isolationskörper zur Verlötung an geeigneten Stellen beispielsweise mit Iridium, Niob, Titan, Tantal oder deren geeignete Legierungen beschichtet.

Zur Beurteilung der von der Durchführung gebildeten hermetischen Abdichtung des Implantat-Inneren gegenüber der Umgebung ist es vorteilhaft, wenn die Bereiche der Sinterverbindungen oder Lötverbindungen (mit Glas-, Glaskeramik oder Goldlot) für einen Helium-Lecktest zugänglich sind und nicht durch einen Filterkörper und dessen elektrisch leitfähige Verbindungen zu den Pins und zum Flansch verdeckt werden. Die Prüfbarkeit der Hermetizität der Durchführung kann auf mehreren Wegen gewährleistet werden:
- Durchgangsöffnung im elektrischen Filterkörper.
- Durchgangsöffnung in einer der elektrisch leitfähigen Verbindungen zwischen dem Filterkörper und den Pins bzw. dem Flansch.
- Der Filterkörper ist in einer Buchse integriert, die über Schweißpunkte mit dem Flansch verbunden ist; der He-Gasdurchtritt ist zwischen den Schweißpunkten gewährleistet.
- Die elektrische Verbindung des Filters mit dem Flansch oder mit den Pins wird durch eine (federnde) Klemmung hergestellt, wobei entweder der Flansch so ausgeformt ist, dass die Federn Bestandteil des Flansches sind oder ein separater Federkörper die elektrische Verbindung zwischen dem Flansch und dem Filter herstellt. Der gewünschte He-Gasdurchtritt erfolgt in diesem Fall zwischen den Klemmpunkten.

In einer Variante sind die Kondensatorelektroden des Filters bereits im Isolationskörper mit integriert, so dass ein separater Filterkörper entfällt. Eine mögliche Ausführungsform ist, dass als Dielektrikum zwischen den Kondensatorelektroden das gleiche keramische Isolationsmaterial (z. B. Al₂O₃) wie an der Oberfläche verwendet wird. In einer weiteren Ausführungsvariante befindet sich ein für die elektrische Filterfunktion angepasstes Material (z. B. BaTiO₃ oder ein anderes keramisches Material hoher Permittivität) zwischen den Kondensatorelektroden, während sich an der Oberfläche ein biokompatibles Isolationsmaterial (z. B. Al₂O₃) befindet.

Um schließlich gleichzeitig eine gute Montierbarkeit und eine gute Dichtigkeit zwischen Flansch und Isolationskörper zu gewährleisten, besitzt der Isolationskörper vorzugsweise einen umlaufenden Absatz in der äußeren Umfangsfläche, der mit einem entsprechenden Absatz in der Innenwand des Flansches zusammenwirkt, wenn die beiden Absätze auf der Umfangsfläche des Isolationskörpers und in der Innenwand des Flansches in Bezug auf die Längsrichtung der Durchführung schräg verlaufen, so dass sich miteinander zusammenwirkende, konische Flächen ergeben, erleichtert der Absatz auch das Zentrieren des Isolationskörpers bezüglich des Flansches.

Die Erfindung soll nun anhand von Ausführungsbeispielen mit Bezug auf die Zeichnungen näher erläutert werden. In den Figuren zeigen:
- Fig. 1:: eine gesinterte Durchführung im Querschnitt; und
- Fig. 2:: einen Herzschrittmacher mit einer erfindungsgemäßen Durchführung.

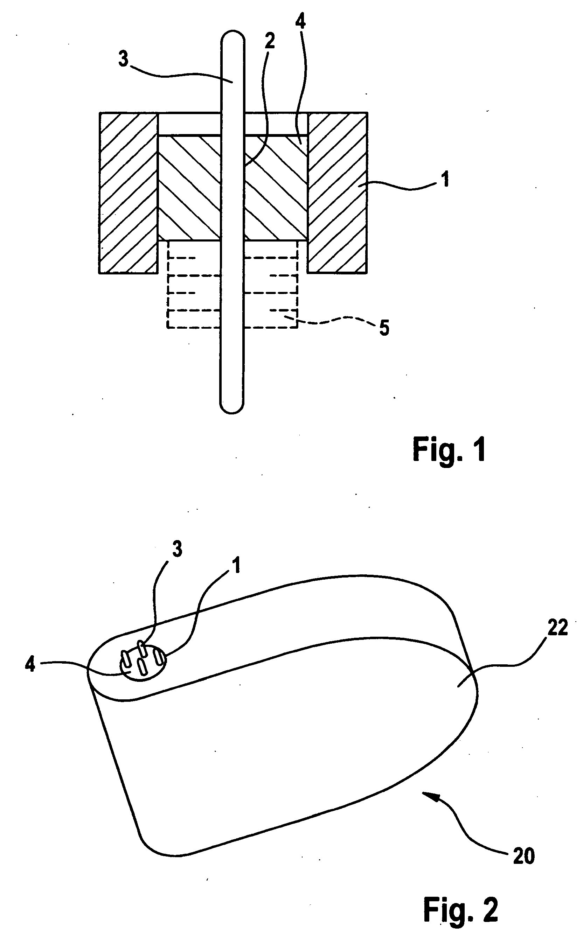

Die in Figur 1 abgebildete Durchführung besitzt einen Flansch 1, der einen Isolationskörper 4 einfasst. Durch den Isolationskörper 4 tritt ein Anschlusspin 3 hindurch. Anstelle eines Anschlusspins können bei mehrpoligen Durchführungen, die hier nicht dargestellt sind, auch mehrere Anschlusspins vorgesehen sein, die vorzugsweise parallel zueinander durch den Isolationskörper 4 hindurchragen.

Zusätzlich kann ein Filterkörper 5 vorgesehen sein, der als Kondensator zwischen dem Flansch 1 und dem Anschlusspin 3 wirkt und auf diese Weise als Tiefpassfilter wirkt, da hochfrequente Störungen mittels des Filterkörpers kurzgeschlossen werden. Der Filterkörper weist hierzu Elektroden auf, die mittels eines elektrisch leitfähigen Verbindungsmaterials, wie beispielsweise ein elektrisch leitfähiges Thermoplast oder ein elektrisch leitfähiges (Metall-) Lot, jeweils abwechselnd mit dem Flansch und mit dem Anschlusspin verbunden sind.

Fig. 1 zeigt beispielhaft eine Durchführung, bei der ein Anschlusspin 3 und ein Isolations-körper 4 sowie ein Flansch 1 durch Sintern miteinander verbunden sind. Der Isolations-körper besteht aus einem isolierenden Material, beispielsweise Glaskeramik oder Keramik, insbesondere Al₂O₃. Durch den Sinterprozess verbindet sich die Glaskeramik oder die Keramik des Isolationskörpers 4 hermetisch dicht mit dem Anschlusspins 3 und dem Flansch 1, so dass jegliches Lot zum Abdichten obsolet ist. Der Anschlusspin 3 besteht aus Metall und kann aus gezogenem Vollmaterial hergestellt sein. Der Flansch 1 kann für sich genommen ebenfalls gesintert sein. In diesem Falle liegen der Flansch 1 und der Isolator 4 vor dem Sinterprozess, der diese Komponenten miteinander verbindet, als gepresster, gespritzter oder anderweitig geformter Grünling vor.

Die durch den Sinterprozess beim Verbinden der Komponenten entstehende Verbindung ist im Ergebnis kraftschlüssig. Vorzugsweise wird der Sinterprozess so durchgeführt, dass es an der Grenzfläche zwischen den Komponenten, insbesondere an der Grenzfläche zwischen Anschlusspin 3 und Isolationskörper 4 einerseits und der Grenzfläche zwischen Isolationskörper 4 und Flansch 1 andererseits zu physikalischen oder chemischen Reaktionen kommt, die sich günstig auf die Langzeitstabilität und die Hermetizität der Durchführung auswirken. Ein Vorteil dieses Herstellungsprozesses ist es, dass er keines weiteren Folgeprozesses bedarf. Ein Vorteil der auf diese Weise hergestellten Durchführung ist es, dass sie besonders dicht und langzeitstabil ist.

Eine Vorbereitung der Grenzflächen, beispielsweise durch Beschichtung oder auf andere Weise, ist in der Regel nicht erforderlich.

Wie in Fig. 1 gestrichelt angedeutet, kann die Durchführung optional einen Filter 5 aufweisen.

Fig. 2 zeigt schließlich beispielhaft einen Herzschrittmacher 20, dessen metallisches Gehäuse bereits mit einer Filterdurchführung der in Fig. 1 abgebildeten Art verschlossen ist. Der Einfachheit halber ist in Fig. 2 nicht der übliche Header eines Herzschrittmachers dargestellt, in dem sich die Anschlussbuchsen für die Elektrodenleitungen befinden. Die elektrischen Kontakte dieser Anschlussbuchsen sind beim fertiggestellten Herzschrittmacher mit den Pins 3 der Filterdurchführung elektrisch leitend verbunden. Die Filterdurchführung - genauer deren Flansch 1 - ist hermetisch dicht mit dem Gehäuse 22 des Herzschrittmachers 20 verbunden, und zwar vorzugsweise durch Verschweißen. Daher ist es vorteilhaft, wenn der Flansch 1 der Filterdurchführung aus dem gleichen Metall besteht, wie das Gehäuse 22 des Herzschrittmachers 20.

## Patentansprüche

1. Elektrische Durchführung zum Einsetzen in eine Öffnung eines implantierbaren elektrischen Therapiegerätes mit einem elektrisch isolierenden Isolationskörper (4; 5), durch den wenigstens ein elektrisch leitender Anschlusspin (3) hindurchtritt, der mit dem Isolationskörper (4; 5) durch Sintern hermetisch dicht verbunden ist, **dadurch gekennzeichnet, dass** die Durchführung einen Flansch (1) aufweist, der in einem Sinterschritt mit dem Isolationskörper (4) und dem mindestes einen Anschlüsspin (3) hermetisch dicht verbunden ist.

2. Durchführung nach Anspruch 1, **dadurch gekennzeichnet, dass** der Isolationskörper (4) aus keramischem, glaskeramischem oder glasartigem Material besteht.

3. Durchführung nach Anspruch 2, **dadurch gekennzeichnet, dass** der Isolationskörper (4) ein Al₂O₃ enthaltender Keramikkörper ist.

4. Durchführung nach einem der Ansprüche 1 bis 3, **dadurch gekennzeichnet, dass** der Anschlusspin (3) oder die Anschlusspins (3) aus Metall bestehen.

5. Durchführung nach Anspruch 4, **dadurch gekennzeichnet, dass** das Metall der Anschlusspins (3) ein Metall aus der Gruppe Platin, Iridium, Niob, Tantal und Titan oder eine Legierung dieser Metalle ist.

6. Durchführung nach Anspruch 5, **dadurch gekennzeichnet, dass** das Metall der Anschlusspins (3) eine Platin-Iridium-Legierung ist.

7. Durchführung nach einem der Ansprüche 1 bis 6, **dadurch gekennzeichnet, dass** die Durchführung zwei oder mehrere Anschlusspins (3) unterschiedlicher Länge aufweist.

8. Durchführung nach einem der Ansprüche 1 bis 7, **dadurch gekennzeichnet, dass** die Durchführung zwei oder mehrere zueinander parallel verlaufende Anschlusspins (3) aufweist.

9. Durchführung nach Anspruch 8, **dadurch gekennzeichnet, dass** die Anschlusspins (3) auf einem konzentrisch zum Isolationskörper (4) verlaufenden Kreisbogen gleichmäßig verteilt sind.

10. Durchführung nach Anspruch 9, **dadurch gekennzeichnet, dass** die Anschlusspins (3) auf einer Gerade oder mehreren parallel zueinander verlaufenden Geraden gleichmäßig verteilt sind.

11. Durchführung nach einem der Ansprüche 1 bis 10, **dadurch gekennzeichnet, dass** eine senkrecht zu einer Längsrichtung des Anschlusspins (3) verlaufende Querschnittsfläche des Isolationskörper (4) rund und vorzugsweise kreisförmig ist.

12. Durchführung nach einem der Ansprüche 1 bis 11, **dadurch gekennzeichnet, dass** der Flansch (1) hülsenartig ausgebildet ist und den Isolationskörper (4) in Bezug auf eine Längsrichtung des Anschlusspins (3) in lateraler Richtung einfasst.

13. Durchführung nach einem der Ansprüche 1 bis 12, **dadurch gekennzeichnet, dass** der Flansch (1) metallisch leitend ist.

14. Durchführung nach Anspruch 13, **dadurch gekennzeichnet, dass** der Flansch (1) aus einem Metall besteht, das weitgehend dem Metall eines Gehäuses eines Therapiegerätes entspricht, für das die Durchführung vorgesehen ist.

15. Durchführung nach Anspruch 13 oder 14, **dadurch gekennzeichnet, dass** der Flansch (1) aus gesintertem Material besteht, welches als Folge des Sinterprozesses zahlreiche Poren enthält.

16. Durchführung nach Anspruch 13, 14 oder 15, **dadurch gekennzeichnet, dass** die Durchführung als Filterdurchführung ausgebildet ist und einen Filterkörper (5) trägt, der Kondensatorelektrodenscheiben (22, 27) aufweist, die abwechselnd mit dem Flansch (1) und mindestens einem der Anschlusspins (3) elektrisch leitend verbunden sind.

17. Durchführung nach einem der Ansprüche 1 bis 16, **dadurch gekennzeichnet, dass** der Isolationskörper (4) einen umlaufenden Absatz (18) in der äußeren Umfangsfläche aufweist.

18. Durchführung nach Anspruch 17, **dadurch gekennzeichnet, dass** der umlaufende Absatz (18) schräg ausgebildet ist.

19. Durchführung nach Anspruch 18, **dadurch gekennzeichnet, dass** der umlaufende schräge Absatz (18) des Isolationskörpers (4) einen korrespondierenden Absatz (19) am Flansch (1) als Zentrierhilfe findet.

20. Durchführung nach Anspruch 19, **dadurch gekennzeichnet, dass** der als Zentrierhilfe für den Isolationskörper (4) dienende Absatz (19) am Flansch (1) passend zum schrägen Absatz (18) des Isolationskörpers (4) schräg ausgebildet ist.

21. Implantierbares Elektrotherapiegerät, insbesondere Herzschrittmacher oder Kardioverter/Defibrillator, mit einer Durchführung gemäß einem der Ansprüche 1 bis 20.

## Claims

1. An electric feedthrough for insertion into an opening in an implantable electric therapeutic device having an electrically insulating insulation body (4; 5) through which at least one electrically conducting connector pin (3) passes and is connected to the insulation body (4; 5) by sintering so that it is hermetically sealed, **characterized in that**
the feedthrough has a flange (1), which is connected to the insulation body (4) and to the at least one connector pin (3) with a hermetic seal in a sintering step.

2. The feedthrough according to Claim 1, **characterized in that** the insulation body (4) is made of a ceramic, glass ceramic or glassy material.

3. The feedthrough according to Claim 2, **characterized in that** the insulation body (4) is a ceramic body comprising Al₂O₃.

4. The feedthrough according to any one of Claims 1 to 3, **characterized in that** the connector pin (3) or the connector pins (3) are made of metal.

5. The feedthrough according to Claim 4, **characterized in that** the metal of the connector pins (3) is a metal from the group of platinum, iridium, niobium, tantalum and titanium or an alloy of these metals.

6. The feedthrough according to Claim 5, **characterized in that** the metal of the connector pins (3) is a platinum-iridium alloy.

7. The feedthrough according to any one of Claims 1 to 6, **characterized in that** the feedthrough has two or more connector pins (3) of different lengths.

8. The feedthrough according to any one of Claims 1 to 7, **characterized in that** the feedthrough has two or more connector pins (3) running parallel to one another.

9. The feedthrough according to Claim 8, **characterized in that** the connector pins (3) are distributed uniformly on an arc of a circle running concentrically with the insulation body (4).

10. The feedthrough according to Claim 9, **characterized in that** the connector pins (3) are uniformly distributed on one straight line or on several straight lines running parallel to one another.

11. The feedthrough according to any one of Claims 1 to 10, **characterized in that** a cross-sectional area of the insulation body (4) running perpendicular to a longitudinal direction of the connector pin (3) is round and preferably circular.

12. The feedthrough according to any one of Claims 1 to 11, **characterized in that** the flange (1) is designed in the form of a sleeve and encompasses the insulation body (4) it in the lateral direction with respect to a longitudinal direction of the connector pin (3).

13. The feedthrough according to any one of Claims 1 to 12, **characterized in that** the flange (1) is metallic and conductive.

14. The feedthrough according to Claim 13, **characterized in that** the flange (1) is made of a metal which largely corresponds to the metal of a housing of a therapeutic device for which the feedthrough is provided.

15. The feedthrough according to Claim 13 or 14, **characterized in that** the flange (1) is made of sintered metal which contains numerous pores as a result of the sintering process.

16. The feedthrough according to Claim 13, 14, or 15, **characterized in that** the feedthrough is designed as a filter feedthrough and has a filter body (5) which has capacitor electrode disks (22, 27) that are connected in an electrically conducting manner to the flange (1) and at least one of the connector pins (3) in alternation.

17. The feedthrough according to any one of Claims 1 to 16, **characterized in that** the insulation body (4) has a circumferential shoulder (18) in the outer circumferential surface.

18. The feedthrough according to Claim 17, **characterized in that** the circumferential shoulder (18) is designed to be inclined.

19. The feedthrough according to Claim 18, **characterized in that** the peripheral inclined shoulder (18) of the insulation body (4) has a corresponding shoulder (19) on the flange (1) as a centering aid.

20. The feedthrough according to Claim 19, **characterized in that** the shoulder (19) which serves as a centering aid for the insulation body (4) is designed at an inclination on the flange (1) so that it fits the inclined shoulder (18) of the insulation body (4).

21. The implantable electrotherapy device, in particular the heart pacemaker or cardioverter/defibrillator, having a feedthrough according to any one of Claims 1 to 20.

## Revendications

1. Passage électrique pour l'insertion dans une ouverture d'un appareil thérapeutique électrique implantable comprenant un corps d'isolation (4 ; 5) électriquement isolant, par lequel passe au moins une broche de raccordement (3) électroconductrice, laquelle est reliée de façon hermétiquement étanche au corps d'isolation (4 ; 5) par frittage, **caractérisé en ce que** le passage présente une bride (1) qui est reliée de façon hermétiquement étanche dans une étape de frittage au corps d'isolation (4) et la broche de raccordement (3) au nombre minimum de un.

2. Passage selon la revendication 1, **caractérisé en ce que** le corps d'isolation (4) est à base de matériau céramique, vitrocérame ou vitreux.

3. Passage selon la revendication 2, **caractérisé en ce que** le corps d'isolation (4) est un corps céramique contenant du Al₂O₃.

4. Passage selon l'une quelconque des revendications 1 à 3, **caractérisé en ce que** la broche de raccordement (3) ou les broches de raccordement (3) sont en métal.

5. Passage selon la revendication 4, **caractérisé en ce que** le métal des broches de raccordement (3) est un métal choisi dans le groupe platine, iridium, niobium, tantale et titane ou un alliage de ces métaux.

6. Passage selon la revendication 5, **caractérisé en ce que** le métal des broches de raccordement (3) est un alliage platine-iridium.

7. Passage selon l'une quelconque des revendications 1 à 6, **caractérisé en ce que** le passage présente deux ou plusieurs broches de raccordement (3) de longueur différente.

8. Passage selon l'une quelconque des revendications 1 à 7, **caractérisé en ce que** le passage présente deux ou plusieurs broches de raccordement (3) agencées parallèlement entre elles.

9. Passage selon la revendication 8, **caractérisé en ce que** les broches de raccordement (3) sont réparties uniformément sur un arc de cercle agencé de façon concentrique par rapport au corps d'isolation (4).

10. Passage selon la revendication 9, **caractérisé en ce que** les broches de raccordement (3) sont réparties uniformément sur une droite ou plusieurs droites agencées parallèlement entre elles.

11. Passage selon l'une quelconque des revendications 1 à 10, **caractérisé en ce qu'**une surface de section, agencée perpendiculairement à une direction longitudinale de la broche de raccordement (3), du corps d'isolation (4) est de forme ronde et de préférence circulaire.

12. Passage selon l'une quelconque des revendications 1 à 11, **caractérisé en ce que** la bride (1) est conçue en forme de manchon et inclut le corps d'isolation (4) par rapport à une direction longitudinale de la broche de raccordement (3) dans le sens latéral.

13. Passage selon l'une quelconque des revendications 1 à 12, **caractérisé en ce que** la bride (1) est conductrice au niveau du métal.

14. Passage selon la revendication 13, **caractérisé en ce que** la bride (1) est à base d'un métal qui correspond largement au métal d'un boîtier d'un appareil thérapeutique, pour lequel le passage est prévu.

15. Passage selon la revendication 13 ou 14, **caractérisé en ce que** la bride (1) est à base de matériau fritté, qui contient de nombreux pores comme conséquence du processus de frittage.

16. Passage selon la revendication 13, 14 ou 15, **caractérisé en ce que** le passage est conçu comme passage de filtre et porte un corps de filtre (5), qui présente des disques d'électrode de condensateur (22, 27), lesquels sont reliés de façon électroconductrice à tour de rôle à la bride (1) et à au moins l'une des broches de raccordement (3).

17. Passage selon l'une quelconque des revendications 1 à 16, **caractérisé en ce que** le corps d'isolation (4) présente un décrochement (18) périphérique dans la surface périphérique extérieure.

18. Passage selon la revendication 17, **caractérisé en ce que** le décrochement (18) périphérique est conçu incliné.

19. Passage selon la revendication 18, **caractérisé en ce que** le décrochement (18) incliné périphérique du corps d'isolation (4) trouve un décrochement (19) correspondant sur la bride (1) comme aide de centrage.

20. Passage selon la revendication 19, **caractérisé en ce que** le décrochement (19) servant d'aide de centrage pour le corps d'isolation (4) est conçu en biais sur la bride (1) de façon adaptée au décrochement (18) incliné du corps d'isolation (4).

21. Appareil d'électrothérapie implantable, en particulier pacemaker ou cardioverteur/défibrillateur, avec un passage selon l'une quelconque des revendications 1 à 20.
